# EUROPEAN PATENT SPECIFICATION

(11) **EP 2 831 058 B1**
(45) Date of publication and mention of the grant of the patent: **24.02.2016**
(21) Application number: 13715911.7
(22) Date of filing: 21.03.2013
(51) Int. Cl.: C07D 401/12, A61K 31/4422, A61P 9/00

(54) **POLYMORPH OF BARNIDIPINE HYDROCHLORIDE AND PROCESSES FOR ITS PREPARATION**
POLYMORPH VON BARNIDIPIN-HYDROCHLORID UND VERFAHREN ZU DESSEN HERSTELLUNG
POLYMORPHE DE CHLORHYDRATE DE BARNIDIPINE ET PROCÉDÉS POUR SA PRÉPARATION

(30) Priority: 29.03.2012 IT MI20120513
(43) Date of publication of application: 04.02.2015
(73) Proprietor: Lusochimica S.p.A., 23871 Lomagna (LC) (IT)
(72) Inventor: POMA, Davide, I-23871 Lomagna (LC) (IT); CAGLIO, Daniele, I-23871 Lomagna (LC) (IT); PORCELLONI, Marina, I-23871 Lomagna (LC) (IT)
(74) Representative: Banfi, Paolo
(86) International application number: PCT/EP2013/055937
(87) International publication number: WO 2013/143967

(56) References cited:
- EP-A2- 0 160 451
- US-A- 4 220 649
- TAMAZAWA K ET AL: "STEREOSELECTIVITY OF A POTENT CALCIUM ANTAGONIST, 1-BENZYL-3-PYRROLIDINYL METHYL 2,6-DIMETHYL-4-(M-NITROPHENYL)- 1,4-DIHYDROPYRIDINE-3,5-DICARBOXYLATE", JOURNAL OF MEDICINAL CHEMISTRY, AMERICAN CHEMICAL SOCIETY, US, vol. 29, no. 12, 1 December 1986 (1986-12-01), pages 2504-2511, XP000564487, ISSN: 0022-2623, DOI: 10.1021/JM00162A013 cited in the application

## Description

### Field of invention

The present invention relates to a novel crystalline form of 3-(3*S*)-1-benzylpyrrolidin-3-yl 5-methyl 2,6-dimethyl-(4*S*)-(3-nitrophenyl)-1,4-dihydropyridine-3,5-dicarboxylate hydrochloride, known as barnidipine hydrochloride, and to processes for its preparation.

### State of the art

Barnidipine hydrochloride is a 1,4-dihydropyridine calcium channel antagonist/blocker, which is widely used to treat hypertension and angina.

Barnidipine hydrochloride of formula (I) [CAS Registry Number 104757-53-1], and the methods for its preparation, were originally disclosed in US 4,220,649, assigned to Yamanouchi Pharmaceutical Co.

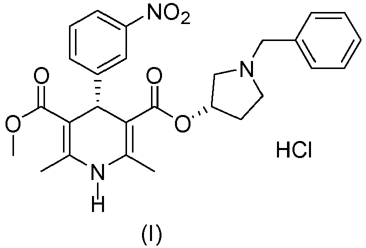

Barnidipine hydrochloride possesses two stereocentres giving rise to 4 different diastereoisomers, described in EP 0 160 451, also assigned to Yamanouchi Pharmaceutical Co, which also reports the procedures for their separation by fractionated crystallisation and/or chromatography.

The description of the procedures for separation and/or resolution of the diastereoisomers, and the crystallographic characterisation of barnidipine hydrochloride (3S,4S), were also subsequently published by the same authors in Journal Medicinal Chemistry, 1986, 29, 2504-2511. Barnidipine hydrochloride is obtained by a coupling reaction between acid IIa and alcohol IIb in the presence of thionyl chloride as activating agent in DMF/DCM, and then crystallised from MEOH to give the desired product.

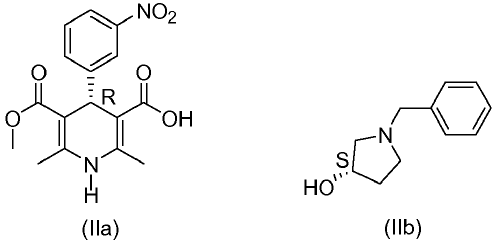

In the article, the product barnidipine hydrochloride (3S,4S) is characterised in terms of melting point (226-228°C), specific rotation ([α]²⁰_{D} (c 1, MeOH) = +116.4°) and, by means of single-crystal X-ray analysis, the three-dimensional structure of the molecule with the corresponding crystallographic parameters. The molecule belongs to spatial group P2₁ with the following dimensions: a = 14.429(4) Å, b = 7.584(2) Å, c = 12.661(4) Å, β = 93.64(2)°, V = 1383 Å³. For the purpose of the present invention, said molecule is called "form I".

The existence of different crystalline forms (polymorphism) is a property of some molecules and molecular complexes. A single molecule can give rise to a variety of solid forms that have distinct physical properties such as their melting point, X-ray diffractogram and infrared absorption. The differences in the physical properties of polymorphs derive from the orientation and intermolecular interactions between adjacent molecules in the solid lattice.

Accordingly, polymorphs are distinct solids having the same molecular formula but physical properties differing from those of the other polymorphs belonging to the same family.

The physical characteristics are influenced by the conformation and orientation of the molecules in the unit cell, which defines the particular polymorphic form of a substance and is unequivocally identified by X-ray spectroscopy. The polymorphic form can give rise to different thermal behaviour from that of the same material in the amorphous form or another polymorphic form. Thermal behaviour is determined by techniques such as melting point, thermogravimetric analysis (TGA) and differential scanning calorimetry (DSC), and can be used to distinguish some polymorphic forms from others. A particular polymorph can also give rise to distinct spectroscopic properties determinable by solid-state NMR analysis and infrared spectroscopy.

Like many other 1,4 dihydropyridine antihypertensives, barnidipine hydrochloride is known to be a photosensitive compound (Talenta 2009, 79, 1418-1424; Journal of Photochemistry and Photobiology A: Chemistry 2010, 215, 205-213; Il Farmaco 2000, 55, 128-133) whose most common photodegradation route is the oxidative process, leading to the corresponding pyridine derivative of formula (III), which does not possess any therapeutic effect.

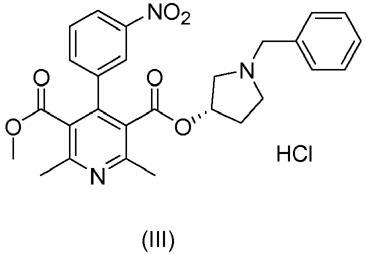

The pharmacologically active ingredient used to prepare the pharmaceutical compositions should be as pure as possible, and its long-term storage stability guaranteed under different environmental conditions.

This is essential to prevent the use of a pharmaceutical composition that contains, in addition to the real active substance, other ingredients such as by-products of oxidation. In these cases, the actual amount of active substance in the final composition of the medicament would be less than specified.

A novel polymorphic form of barnidipine hydrochloride, called "form II" for the purpose of the present invention, has now surprisingly been found. This provides a new opportunity to improve and facilitate the handling and storage of barnidipine hydrochloride as active ingredient, because it is a light-stable crystalline form.

### Summary of the invention

The object of this invention is a novel, more thermodynamically stable form of barnidipine hydrochloride, called Form II.

Crystalline form II of barnidipine hydrochloride is characterised by an orthorhombic cell, spatial group P2₁2₁2₁, with the following dimensions: a = 7.185(2) Å, b = 16.123(4) Å, c = 22.571(6) Å, V = 2614.7(12) Å³.

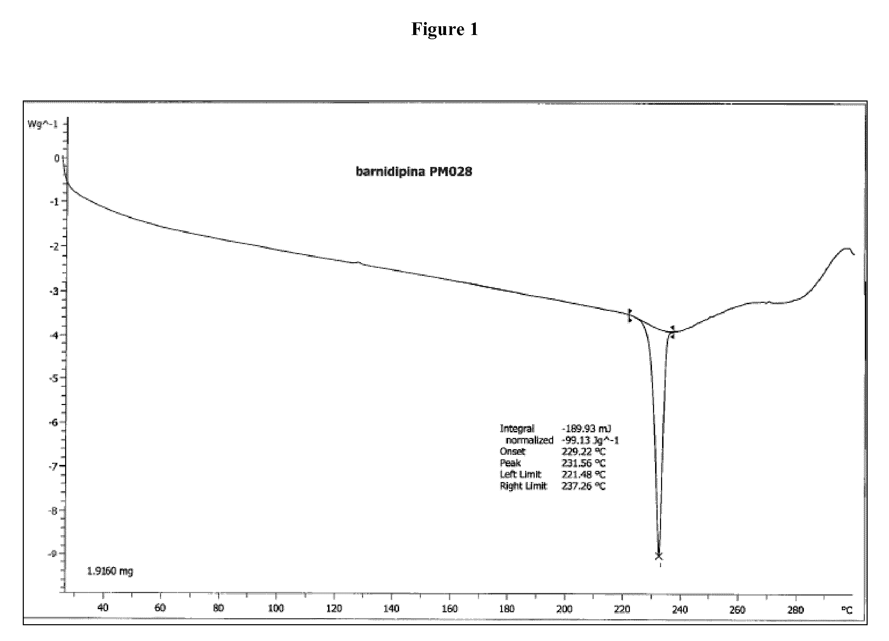

Form II has the DSC diagram shown in Figure 1, characterised by a narrow endothermic peak at 229.2°C in the DSC diagram.

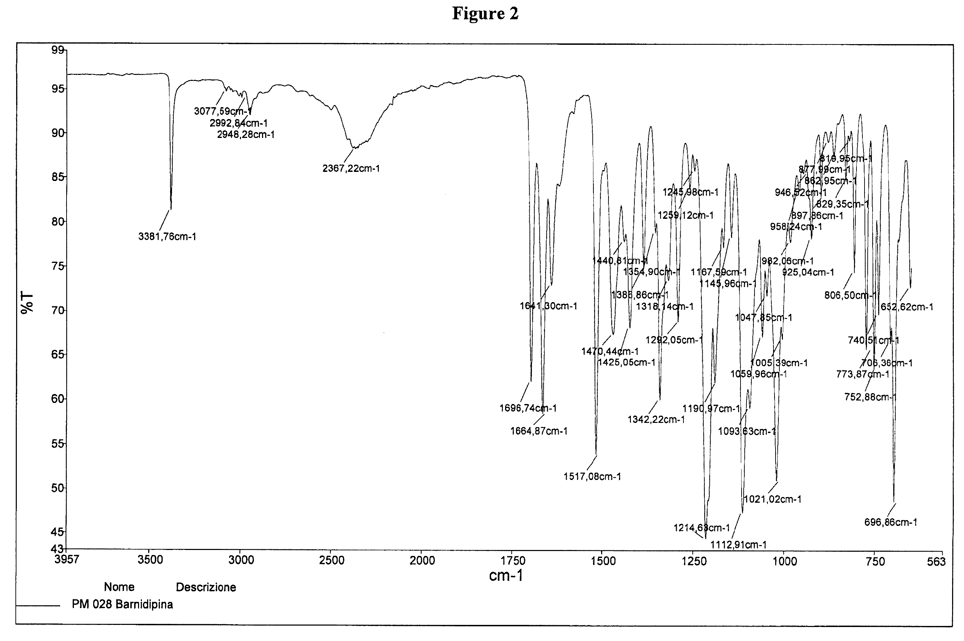

Form II has the IR spectrum shown in Figure 2, characterised by a system of main IR peaks at 3382, 2948, 1665, 1517, 1425, 1292 and 1191 cm-¹.

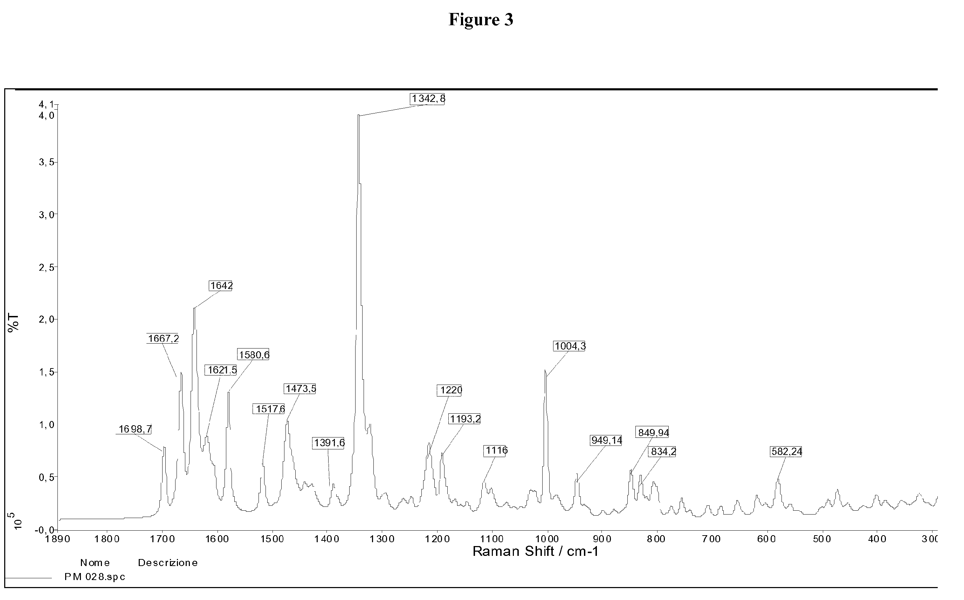

Form II also has the Raman spectrum shown in Figure 3, characterised by a system of main Raman peaks at 1699, 1642, 1517, 1342, 1193, 1004, 849 and 582 cm⁻¹.

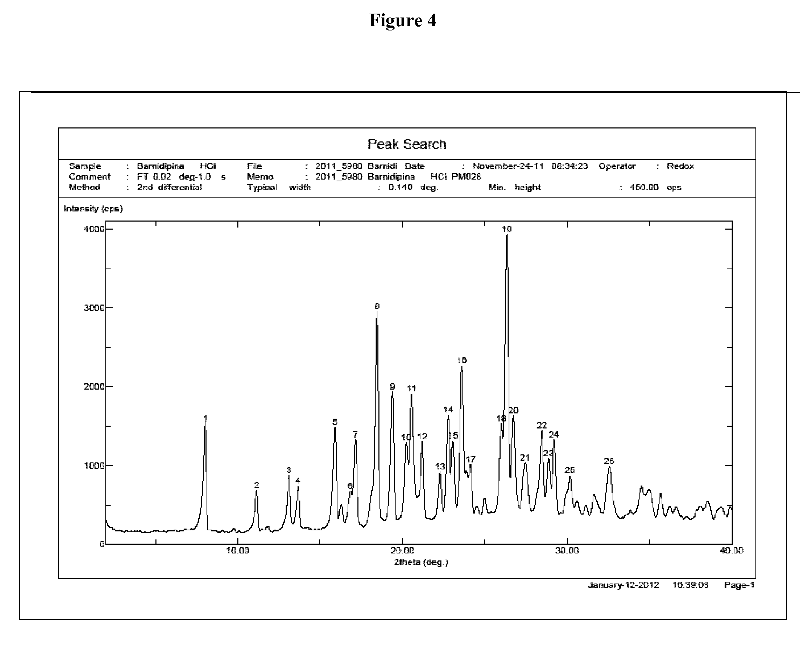

Finally, crystalline form II of barnidipine hydrochloride has the X-ray powder diffractogram (XRPD) shown in Figure 4, characterised by a system of main diffraction peaks expressed in 2-theta degrees [°] at 8.0 ±0.2, 13.01±0.2, 18.4±0.2 and 24.1 ±0.2.

Another object of the present invention is a method for obtaining form II of barnidipine hydrochloride by means of crystallisation of crude barnidipine hydrochloride by heating it to between 60° and 90°C in a suitable organic solvent.

### Brief description of figures

Figure 1: DSC diagram of barnidipine hydrochloride, form II
Figure 2: FTIR spectrum of barnidipine hydrochloride, form II
Figure 3. Raman spectrum of barnidipine hydrochloride, form II
Figure 4: XRPD diffractogram of barnidipine hydrochloride, form II
Figure 5. DSC diagram of barnidipine hydrochloride, form I
Figure 6. FTIR spectrum of barnidipine hydrochloride, form I
Figure 7. Raman spectrum of barnidipine hydrochloride, form I
Figure 8. XRPD diffractogram of barnidipine hydrochloride, form I
Figure 9. X-ray powder diffractogram calculated in the 2θ=5-50° range for barnidipine hydrochloride (λ=1.54056 Å) from the literature, obtained with PowderCell, compared with the diffractogram recorded for barnidipine hydrochloride form I.

### Detailed description of the invention

Crude barnidipine hydrochloride of formula (I) can be obtained according to known methods by treatment with hydrochloric acid of the free base, which in turn is obtained by basic treatment of the corresponding salt (US 4,220,649, JMC, 1986, 29, 2504-2511).

It has now been found that, depending on the purification conditions of the crude product, barnidipine hydrochloride can be obtained in two distinct polymorphic forms, herein called form I and form II.

Polymorphic form I of barnidipine hydrochloride is the form known to the skilled person which is cited in the literature (Journal Medicinal Chemistry 1986, 29, 2504-2511), and is very pale yellow / white.

However, polymorphic form II of barnidipine hydrochloride, which is the object of this invention, has a deep yellow colour.

The two different polymorphic modifications are selectively obtainable by suitably adjusting the conditions applied in the crystallisation process.

It has also been found that the two different polymorphs can be interconverted from Form I to Form II and vice versa by applying suitable recrystallisation conditions.

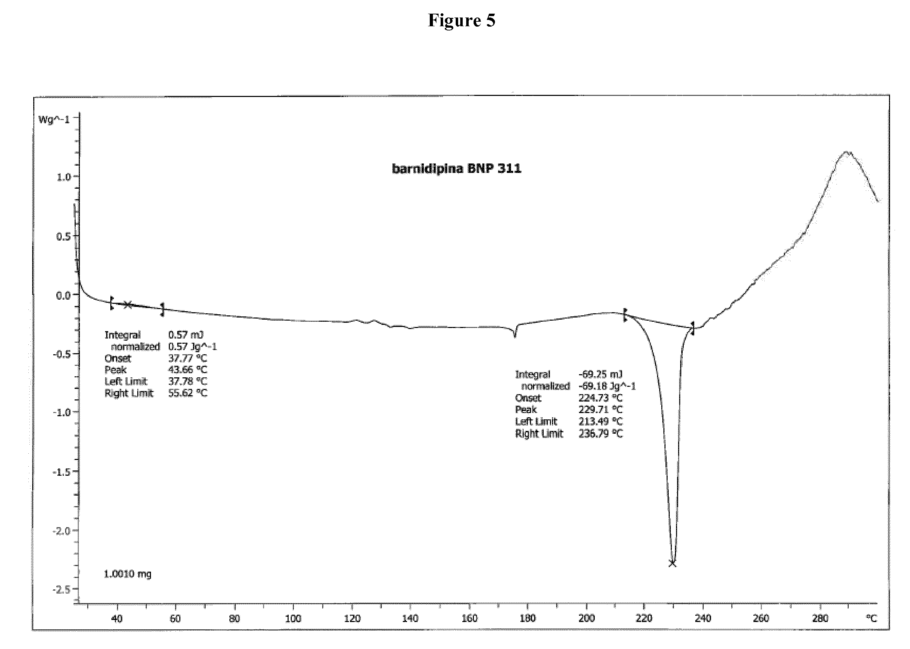

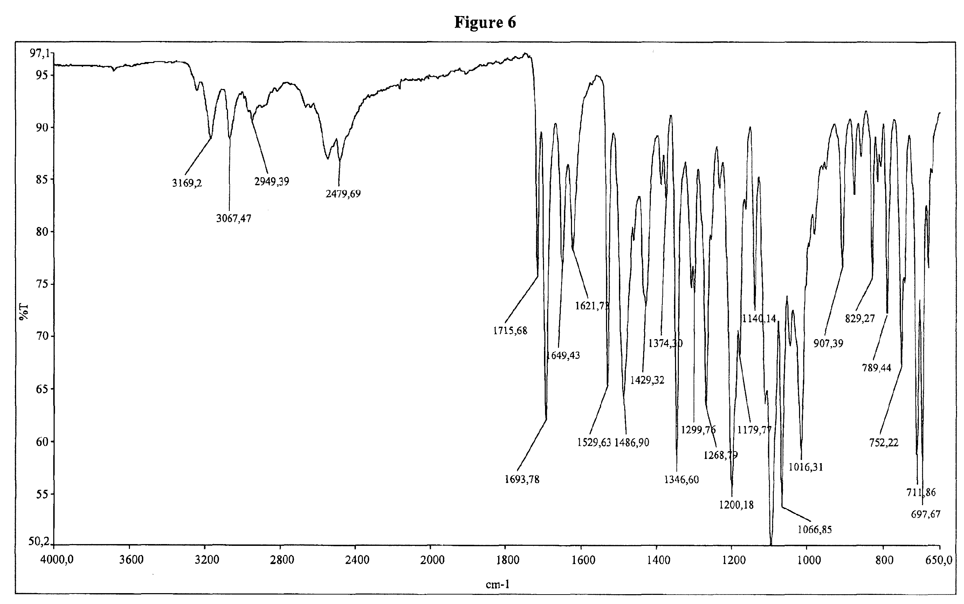

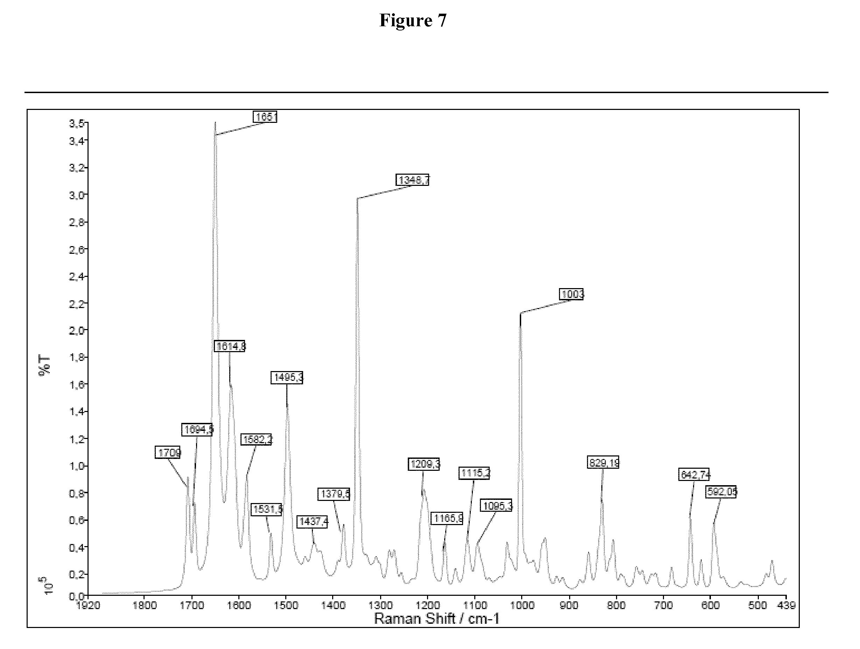

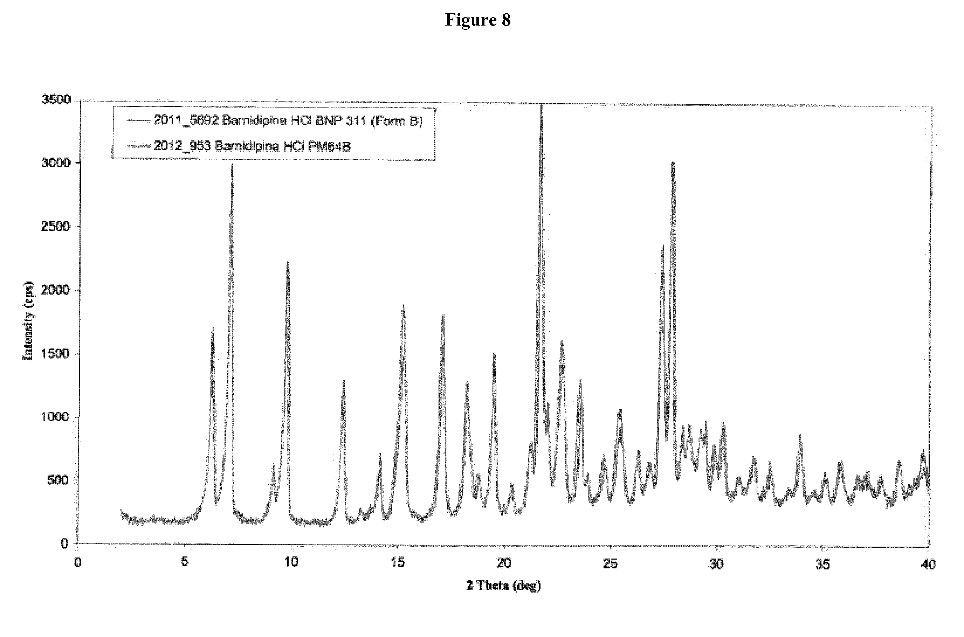

In particular, by operating as described in J. Med. Chem., 1986, 29, 2504-2511, a pale yellow barnidipine hydrochloride with the following characteristics is obtained:
- a DSC diagram as shown in Figure 5, characterised by a narrow endothermic peak at 230.13°C
- an IR spectrum as shown in Figure 6, characterised by a system of main IR peaks at 3242-2949-1715-1648-1622-1487-1429-1346-1269-1200-1066-807-697 cm-¹
- a Raman spectrum as shown in Figure 7, characterised by a system of main Raman peaks at 1695-1651-1614-1349-1209-1003-829, cm⁻¹
- an XRPD diffractogram as shown in Figure 8, characterised by a system of main peaks expressed in 2-theta degrees [°] at 6.2±0.2, 7.1±0.2, 9.7±0.2, 15.2±0.2, 21.7 ±0.2.

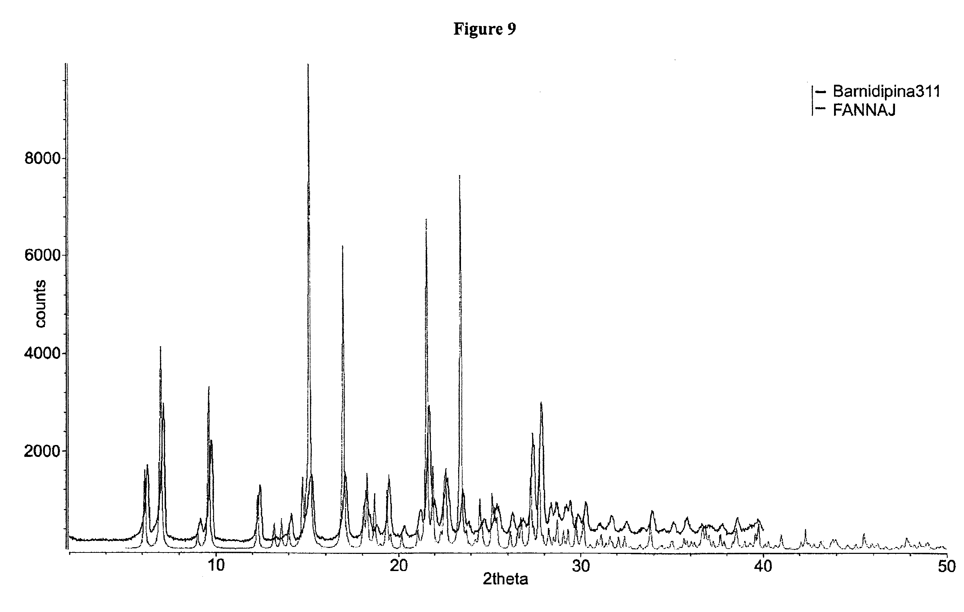

On the basis of the crystallography data published in J.M.C., 1996, 29, 2504-2511, and the references cited herein, relating to barnidipine hydrochloride produced according to the state of the art, an X-ray powder simulation was conducted with PowderCell (for Windows, version 2.4). The diffractogram obtained from said simulation, overlaid on the one obtained experimentally with our sample of barnidipine hydrochloride, showed a very high correlation (Figure 9), and the small variations observed can be attributed to the low resolution in the experimental acquisition compared with the diffraction lines calculated.

This clearly demonstrates that the product we obtained by following the indications in the literature corresponds to form I, known to the prior art.

It is also evident that the form I of barnidipine hydrochloride obtained differs considerably from the other form we obtained and called form II, in terms of all the chemico-physical properties analysed (DSC, IR, Raman and XRPD diffractogram).

Specifically, it has been found that polymorphic form II of barnidipine hydrochloride can be obtained in pure crystalline form, and is the most thermodynamically stable form of the molecule.

Crystalline form II of barnidipine hydrochloride is characterised by an orthorhombic cell, spatial group P2₁2₁2₁, with the following dimensions: a = 7.185(2) Å, b = 16.123(4) Å, c = 22.571(6) Å, V = 2614.7(12) Å³.

Form II presents the DSC diagram shown in Figure 1, characterised by a narrow endothermic peak at 229.2±2°C in the DSC diagram.

Form II has the IR spectrum shown in Figure 2, characterised by a system of main IR peaks at 3382, 2948, 1665, 1517, 1425, 1292 and 1191 cm⁻¹.

Form II also has the Raman spectrum shown in Figure 3, characterised by a system of main Raman peaks at 1699, 1642, 1517, 1342, 1193, 1004, 849 and 582 cm⁻¹.

Finally, crystalline form II presents the X-ray powder diffractogram (XRPD) shown in Figure 4, characterised by a system of main diffraction peaks expressed in 2-theta degrees [°] at 8.0 ±0.2, 13.6±0.2, 18.4±0.2 and 24.1 ±0.2. The peaks at 11.1±0.2, 17.1±0.2, 22.2±0.2 and 26.7±0.2 constitute an additional group of characterising diffraction peaks expressed in 2-theta degrees [°]. The peaks at 13.1±0.2, 16.8±0.2, 22.7±0.2 and 30.1±0.2 constitute a further group of characterising diffraction peaks expressed in 2-theta degrees [°].

Another object of this invention is a new process for obtaining form II of barnidipine hydrochloride by means of crystallisation of crude barnidipine hydrochloride by heating it to between 60° and 90°C in a suitable organic solvent.

In the preparation, crude barnidipine hydrochloride, obtained by methods known in the literature (US 4,220,649, JMC, 1986, 29, 2504-2511), is dissolved in an organic solvent, such as ethanol, under stirring at the reflux temperature or in any event at temperatures exceeding 60°C, preferably exceeding 70°C, until the product has completely dissolved.

Part of the solvent is removed by distillation until the incipient formation of a precipitate; the resulting suspension is maintained at a temperature between 60°C and 90°C, preferably between 70°C and 85°C, or in any event at the reflux temperature of the solvent when said temperature is in the range between 60°C and 90°C, for 3 to 20 hours, preferably 5 to 18 hours, and most preferably between 8 and 14 hours, until the precipitation of barnidipine hydrochloride form II. The mixture is then left to cool at ambient temperature for between 1 and 4 hours, preferably between 2 and 4 hours, and more preferably for 2.5 hours, to complete the crystallisation.

Crystallisation can be performed in different solvents, selected from the group comprising ethanol, isopropyl alcohol, propanol, acetone, methyl ethyl ketone, acetonitrile, mixtures thereof and mixtures between them and water, the solvents in the group comprising ethanol, acetone, acetone/water and ethanol/water being preferred, and ethanol being particularly preferred.

The term "ethanol" is used to mean one of the forms in which it is commonly found, selected from pure ethanol (95% purity), absolute ethanol (100% purity) and denatured ethanol (>90% purity). Denatured ethanol containing 3-6% methanol and 1-3% cyclohexane was also used for the purpose of the present invention.

A typical example of preparation of barnidipine hydrochloride, form II, is as follows.

In a suitable reaction vessel, 0.1 moles of crude barnidipine hydrochloride, obtained in accordance with methods known to the prior art, is suspended in ethanol in an amount ranging from 0.67 kg to 1.0 kg, preferably from 0.75 kg to 0.92 kg, and more preferably 0.83 kg.

The resulting mixture is heated under stirring at a temperature exceeding 60°C, and preferably exceeding 70°C. The maximum operating temperature is determined by the boiling point of the solvent system used. Heating causes the product to dissolve completely.

Part of the solvent, preferably 0.33 kg to 0.66 kg, and more preferably 0.5 kg, is removed by distillation, until barnidipine hydrochloride precipitates.

The suspension is then left under stirring, heated to a temperature between 60°C and 90°C, preferably between 70°C and 85°C, and most preferably at the reflux temperature of ethanol at 78°C, for 3 to 20 hours, preferably 5 to 18 hours, and more preferably 8 to 14 hours. The mixture is then left to cool at ambient temperature for between 1 and 4 hours, preferably between 2 and 4 hours, and more preferably for 2.5 hours, to complete the crystallisation. The crystals obtained are isolated by filtration, washing with 0.05 kg to 0.10 kg, and preferably 0.08 kg, of ethanol.

Barnidipine hydrochloride form I, which constitutes the reference compound, can be prepared from crude barnidipine hydrochloride obtained according to the prior art (US 4,220,649, JMC, 1986, 29, 2504-2511). It is dissolved hot in an organic solvent, for example in methanol or ethanol, cooled and left under stirring for 3 hours until barnidipine hydrochloride form I is obtained.

A typical preparation example of barnidipine hydrochloride form I, prepared as reference compound, is reported here.

0.1 mol of crude barnidipine hydrochloride is suspended in 0.67 - 0.83 kg of ethanol; the resulting mixture is then heated under stirring to a temperature exceeding 60°C, generally to boiling point, until the product has completely dissolved.

The solution obtained is then left to cool slowly in 1-3 hours, under stirring, to ambient temperature or lower, up to 0°C (ice bath), and left at this temperature for 1-4 hours, until precipitation is complete. The crystals obtained are isolated by filtration, washing with 0.05 - 0.10 kg of ethanol.

Form I of barnidipine hydrochloride can be obtained, under suitable conditions, in solvents or mixtures thereof chosen from methanol, ethanol, acetone/water and ethanol/water.

### Experimental part

### Characteristics of barnidipine hydrochloride form II

### DSC

Form II of barnidipine hydrochloride, obtained in accordance with the method described, was analysed by differential scanning calorimetry (DSC). The DSC diagram, recorded at a heating rate set at 10°C/min under nitrogen flow at 30 mL/min, shows a characteristic narrow endothermic peak at 229.2°C which can be associated with a melting point of the compound between 221 and 237°C (Figure 1). These data were obtained with a Mettler-Toledo 821 instrument, by depositing the sample in a hermetically sealed aluminium container.

### IR spectroscopy

Form II of barnidipine hydrochloride was characterised by FTIR/ATR spectroscopy (Fourier Transform Infra Red Spectroscopy, with Attenuated Total Reflectance). The data were obtained with a Perkin Elmer Spectrum Two instrument with diamond probe, with air as background and a resolution of 4 cm⁻¹

The IR spectrum was recorded in solid phase. It was found that form II of barnidipine hydrochloride has substantially the same IR spectrum as illustrated in Figure 2. The characteristic bands of the IR spectrum and the corresponding assignments are listed in Table 1.

The IR spectrum reported in Figure 2 presents main bands at wave numbers 3382, 2948, 1697, 1665, 1641, 1517, 1470, 1425, 1355, 1342, 1292, 1214, 1191, 1113, 1060 and 1021 cm⁻¹ *inter alia.*

**Table 1. FTIR form II: list of characteristic bands and their assignment**

| **Band (cm⁻¹)** | **Assignment** |
|---|---|
| 3382 | Upper stretching>C=O |
| 2948 | Stretching CH₂ |
| 1697 | Stretching >C=O |
| 1665 | Stretching -CH ring |
| 1641 | |
| 1517 | -NH ring |
| 1470 | Tertiary N-C |
| 1425 | |
| 1355 | Stretching NO₂ |
| 1342 | Stretching CH₃ |
| 1292 | Stretching -CN ring |
| 1214 | Stretching -C-O-C- |
| 1113 | Stretching CN |
| 1021 | Wagging CH |

### Raman spectroscopy

Form II of barnidipine hydrochloride was characterised in Raman spectroscopy using a Kaiser Optical RXN2 Hybrid instrument with laser probe and air as background. The morphology of the sample was estimated with a SEM (Scanning Electron Microscope) FEI S50 instrument with particle accelerator having a voltage of 10 kV supported on an adhesive graphite plate covered with gold leaf. The characteristic bands of the Raman spectrum and their assignments are listed in Table 2.

The Raman spectrum reported in Figure 3 presents the main bands at 1699, 1667, 1642, 1622, 1581, 1517, 1474, 1342, 1220, 1193, 1116, 1004, 949, 849, 834 and 582 cm⁻¹.

**Table 2. Raman spectrum: list of characteristic bands and their assignment**

| **Raman shift (cm⁻¹)** | **Assignment** |
|---|---|
| 1699 | >C=O |
| 1667 | C=C |
| 1642 | O-C=O |
| 1622 | -NH₂ |
| 1342 | -CH₃ |
| 1220 | -CN |
| 1004 | -C-O |
| 949 | -C-O |
| 849 | -C-O-C- |
| 834 | |

### XRPD diffractogram

Form II of barnidipine hydrochloride was characterised by structural X-ray powder diffractometry (XRPD) analysis. The X-ray powder diagram was recorded with the MINIFLEX (Rigaku) instrument by depositing the sample on a static support with goniometric radius (distance between the X-ray tube and the support or, the equivalent, between the support and the detector) of 150 mm. The sample of barnidipine hydrochloride, form II, exhibited the XRPD spectrum with the main bands listed in Table 3.

**Table 3: XRPD: list of characteristic peaks**

| **2 Θ [°]** | **d-value [Å]** | **Intensity** | **I/Io** |
|---|---|---|---|
| 8.02 | 11.01 | 1523 | 39 |
| 11.16 | 7.92 | 684 | 18 |
| 13.10 | 6.75 | 874 | 23 |
| 13.66 | 6.47 | 738 | 19 |
| 15.90 | 5.56 | 1486 | 38 |
| 16.84 | 5.26 | 674 | 18 |
| 17.14 | 5.17 | 1327 | 34 |
| 18.46 | 4.80 | 2953 | 76 |
| 19.40 | 4.57 | 1936 | 50 |
| 20.22 | 4.39 | 1296 | 33 |
| 20.54 | 4.32 | 1906 | 49 |
| 21.20 | 4.18 | 1306 | 34 |
| 22.28 | 3.99 | 922 | 24 |
| 22.78 | 3.90 | 1637 | 42 |
| 23.06 | 3.85 | 1309 | 34 |
| 23.60 | 3.77 | 2265 | 58 |
| 24.14 | 3.68 | 1010 | 26 |
| 26.00 | 3.42 | 1529 | 39 |
| 26.34 | 3.38 | 3936 | 100 |
| 26.74 | 3.33 | 1629 | 42 |
| 27.46 | 3.24 | 1035 | 27 |
| 28.46 | 3.13 | 1436 | 37 |
| 28.86 | 3.09 | 1093 | 28 |
| 29.20 | 3.05 | 1327 | 34 |
| 30.16 | 2.96 | 871 | 23 |
| 32.56 | 2.75 | 985 | 26 |

In Table 3, the value "2Θ [°]" represents the angle of diffraction in degrees, and "d-value [Å]" represents the specific intervals between the lattice planes in Å.

The XRPD diffractogram of form II is shown in Figure 4.

It is notable that the pattern for crystalline form II is unique, exhibiting a unique set of diffraction peaks which can be expressed as 20 angle (°) and d-value (Å) and secondarily, as the relative intensity of the peaks. Wide variations in relative intensity can be observed due to the different preferential orientations resulting from the morphological differences of the crystal. Identification of the exact crystalline form of a compound should be based on observation of the 20 angles and/or value d.

However, if both parameters (the 20 angles and values of d-value) are taken into consideration, the X-ray powder diffractogram (XRPD) shown in Figure 4 is characterised by a system of main diffraction peaks expressed in 2-theta degrees [°] at 8.0 ±0.2, 11.2±0.2, 13.1±0.2, 13.7±0.2 and 15.9±0.2. The peaks at 18.5±0.2, 20.2±0.2, 26.0±0.2 and 28.9±0.2 constitute an additional group of characterising diffraction peaks expressed in 2-theta degrees [°]. The peaks at 16.8±0.2, 20.5±0.2, 24.1±0.2 and 30.2±0.2 constitute a further group of characterising diffraction peaks expressed in 2-theta degrees [°].

Globally, form II of barnidipine hydrochloride is consequently characterised by an X-ray powder diagram with characteristic values of 2Θ = 8.0±0.2°, 11.2±0.2°, 13.1±0.2°, 13.7±0.2°, 15.9±0.2°, 16.8±0.2°, 17.1±0.2°, 18.5±0.2°, 19.4±0.2°, 20.2±0.2°, 20.5±0.2°, 21.2±0.2°, 22.3±0.2°, 22.8±0.2°, 23.1±0.2°, 23.6±0.2°, 24.1±0.2°, 26.0±0.2°, 26.3±0.2°, 26.7±0.2°, 27.5±0.2°, 28.5±0.2°, 28.9±0.2°, 29.2±0.2°, 30.2±0.2° and 32.6 ±0.2° and d-value = 11.01 Å, 7.92 Å, 6.75 Å, 6.47 Å, 4.80 Å, 3.85 Å, 3.38 Å and 3.24 Å *inter alia.*

### Single-crystal diffraction

The molecular and crystalline structure of polymorphic form II of barnidipine hydrochloride was determined by single-crystal X-ray diffraction analysis. A single needle-shaped crystal of barnidipine hydrochloride was deposited on fibreglass, and the diffraction values were recorded using MoKα (λ=0.71073 Å) radiation in an APEX2 CCD diffractometer at ambient temperature (293K), applying Lorentz polarisation and absorption corrections. The structure was resolved by direct methods using the SIR2004 programme, and redefined with an FMLQ (full-matrix least-squares) method on the whole F² using the SHELXL97 programme implemented in the WingX package. The experimental details of the crystalline structural resolution are listed in Table 4.

**Table 4. Crystallography data and structural adaptations for barnidipine hydrochloride, form II**

| | |
|---|---|
| Empirical formula | C₂₇H₃₀ClN₃O₆ |
| Formula weight | 527.99 |
| Temperature/K | 293(2) |
| Crystalline system | Orthorhombic |
| Spatial group | P2₁2₁2₁ |
| a/Å | 7.185(2) |
| b/Å | 16.123(4) |
| c/Å | 22.571(6) |
| Volume/Å³ | 2614.7(12) |
| Z | 4 |
| ρ_{calc}mg/mm³ | 1.341 |
| m/mm⁻¹ | 0.193 |
| F(000) | 1112.0 |
| 2Θ data collection interval | 3.1 to 44.06° |
| Index intervals | -7 ≤ h ≤ 7, -17 ≤ k ≤ 17, -23 ≤ 1 ≤ 23 |
| No. of reflections | 19015 |
| Independent reflections | 3212[R(int) = 0.0760] |
| Data/restraints/parameters | 3212/0/346 |
| Accuracy on F² | 1.105 |
| Final R indexes [I>=2σ (I)] | R₁ = 0.0525, wR₂ = 0.1312 |
| Final R indexes [all data] | R₁ = 0.0573, wR₂ = 0.1338 |
| Width ΔFmax/min/ and Å⁻³ | 0.31/-0.32 |

Structural X-ray analysis has demonstrated that form II of barnidipine hydrochloride belongs to the orthorhombic crystalline system, spatial group P2₁2₁2₁, with the following dimensions: a = 7.185(2) Å, b = 16.123(4) Å, c = 22.571(6) Å, V = 2614.7(12) Å³.

The atomic coordinates determined by the structural analysis described above are listed in Table 5.

**Table 5. Atomic coordinates (×104) and equivalent isotropic displacement parameters (Å2×10³) for barnidipine hydrochloride. U(eq) is defined as 1/3rd of the trace of orthogonal tensor UIJ**

| **Atom** | **x** | **y** | **z** | **U(eq)** |
|---|---|---|---|---|
| Cl | 2901.4(18) | 9948.0(8) | 9873.9(6) | 65.7(4) |
| N1 | 1901(6) | 5666(2) | 7768.7(19) | 48.3(10) |
| N2 | 2363(5) | 8916(2) | 7246.7(17) | 45(1) |
| N3 | 7235(6) | 10015(2) | 9864.7(17) | 53.8(10) |
| O1 | 4729(6) | 7873(3) | 5665.8(15) | 74.8(11) |
| O2 | 7029(4) | 7462(2) | 6249.9(14) | 60.8(9) |
| O3 | 732(5) | 5950(2) | 7429.8(18) | 67.8(10) |
| O4 | 1693(5) | 5026(2) | 8059.2(16) | 66.6(10) |
| O5 | 8314(4) | 8767.2(19) | 8126.2(15) | 52.5(8) |
| O6 | 6366(4) | 9774.1(17) | 8425.3(12) | 41.3(7) |
| C1 | 5799(5) | 8125(2) | 7267.6(17) | 33.3(10) |
| C2 | 4675(6) | 8223(2) | 6699.8(18) | 38.1(10) |
| C3 | 2930(6) | 8566(2) | 6721.8(19) | 39.5(10) |
| C4 | 3522(6) | 9135(2) | 7706.8(19) | 36.6(10) |
| C5 | 5305(5) | 8819(2) | 7702.8(17) | 33.2(10) |
| C6 | 5402(6) | 7856(3) | 6151(2) | 43.8(11) |
| C7 | 7873(8) | 7042(4) | 5750(2) | 72.1(16) |
| C8 | 1574(7) | 8635(3) | 6220(2) | 58.9(14) |
| C9 | 5564(5) | 7276(2) | 7553.6(18) | 33.7(10) |
| C10 | 7035(6) | 6902(3) | 7860(2) | 44.7(11) |
| C11 | 6834(7) | 6139(3) | 8137(2) | 53.7(13) |
| C12 | 5149(7) | 5730(3) | 8120(2) | 48.8(12) |
| C13 | 3714(6) | 6105(3) | 7813.0(19) | 40.3(11) |
| C14 | 3861(6) | 6857(3) | 7538.2(19) | 38.5(10) |
| C15 | 6779(6) | 9089(3) | 8103.1(18) | 36.2(10) |
| C16 | 7787(6) | 10084(3) | 8822.6(18) | 41.3(10) |
| C17 | 7093(7) | 10919(3) | 9051(2) | 50.6(12) |
| C18 | 7475(7) | 10899(3) | 9712.2(19) | 51.0(12) |
| C19 | 7986(8) | 9543(3) | 9367.4(19) | 50.6(12) |
| C20 | 7859(8) | 9789(3) | 10464.6(19) | 53.7(13) |
| C21 | 7689(7) | 8877(3) | 10596.5(19) | 49.6(12) |
| C22 | 9205(9) | 8356(4) | 10552(3) | 75.0(17) |
| C23 | 9006(14) | 7522(5) | 10672(4) | 106(3) |
| C24 | 7269(17) | 7214(5) | 10849(3) | 112(3) |
| C25 | 5739(11) | 7734(4) | 10902(3) | 88(2) |
| C26 | 5964(9) | 8556(4) | 10776(2) | 69.1(16) |
| C27 | 2648(6) | 9718(3) | 8143(2) | 49.7(12) |

It has been found that polymorphic form II of barnidipine hydrochloride is stable after at least one month's exposure to sunlight and/or artificial light. HPLC studies have demonstrated that after one month's exposure to sunlight under the environmental conditions of our manufacturing site in Lomagna (Italy), barnidipine hydrochloride form II in the solid state does not undergo degradation of any kind, and in particular does not undergo oxidation leading to the formation of the impurity of formula III. Table 6 contains a selection of the results of the HPLC analyses obtained by analysing the samples of the two crystalline forms I and II of barnidipine hydrochloride exposed to light. As will be seen, form I exhibits substantial oxidative degradation with formation of the impurity of formula III after only two hours' exposure. Moreover, the formation of other degradation products can always be observed in the chromatograms, albeit in smaller amounts. However, in the chromatogram relating to barnidipine hydrochloride form II, no formation of any impurity was observed after one month's exposure to ambient light. Moreover, whereas a definite change in colour from very pale yellow/white to ochre is observed for form I after only two hours, the deep yellow colour of form II remains unchanged even after a month's exposure to light.

**Table 6**

| **Barnidipine hydrochloride** | **Time** | **% impurity of formula III** |
|---|---|---|
| Form I | 0 | 0.02 |
| Form I | 2 hours | 1.24 |
| Form I | 5 hours | 2.03 |
| Form I | 3 days | 9.73 |
| Form I | 1 month | 10.25 |
| Form II | 0 | nd |
| Form II | 2 hours | nd |
| Form II | 3.5 hours | nd |
| Form II | 5 hours | nd |
| Form II | 3 days | nd |
| Form II | 1 month | nd |

### Characterisation of barnidipine hydrochloride, form I

### DSC

Barnidipine hydrochloride, form I, obtained according to the method described above, was analysed by differential scanning calorimetry (DSC) recorded at a heating rate set at 10°C/min under nitrogen flow at 30 mL/min. The DSC diagram shown in Figure 5 exhibits a characteristic narrow endothermic peak at 230.1 °C, which can be associated with a melting point of the compound between 213-237°C (Figure 5). These data were obtained with a Mettler-Toledo 821 instrument, by depositing the sample in a hermetically sealed aluminium container.

### IR spectrum

Form I of barnidipine hydrochloride was characterised by FTIR/ATR spectroscopy (Fourier Transform Infra Red Spectroscopy, with Attenuated Total Reflectance). The data were obtained with a Perkin Elmer Spectrum Two instrument with diamond probe, with air as background and a resolution of 4 cm⁻¹.

The IR spectrum was recorded in solid phase. Form I of barnidipine hydrochloride has the IR spectrum illustrated in Figure 6, while the characteristic bands with the corresponding assignments are listed in Table 7.

**Table 7. FTIR form I: list of characteristic bands and their assignment**

| **Band (cm⁻¹)** | **Assignment** |
|---|---|
| 3242-3069 | Upper stretching >C=O |
| 2949 | Stretching CH₂ |
| 1715 | C=O-O- |
| 1694 | Stretching >C=O |
| 1648 | Stretching -CH ring |
| 1622 | |
| 1529 | -NH ring |
| 1487 | Tertiary N-C |
| 1428 | |
| 1346 | Stretching -CH₃ |
| 1269 | Stretching -CN ring |
| 1200 | Stretching -C-O-C- |
| 1113 | Stretching CN |
| 1095 | Wagging CH |

### Raman spectrum

Form I of barnidipine hydrochloride was characterised by Raman spectroscopy using a Kaiser Optical RXN2 Hybrid instrument with laser probe and air as background. The morphology of the sample was estimated with a SEM (Scanning Electron Microscope) FEI S50 instrument with particle accelerator having a voltage of 10 kV supported on an adhesive graphite plate covered with gold leaf. The characteristic bands of the Raman spectrum and their assignments are listed in Table 8.

**Table 8. Raman spectrum: list of characteristic bands and their assignment**

| **Raman shift (cm⁻¹)** | **Assignment** |
|---|---|
| 1695 | >C=O |
| 1651 | C=C |
| 1614 | -NH₂ |
| 1349 | -CH₃ |
| 1209 | -CN |
| 1003 | -C-O |
| 829 | -C-O-C- |

The Raman spectrum is reported in Figure 7

### XRPD diffractogram

Form I of barnidipine hydrochloride was characterised by structural X-ray powder diffractometry (XRPD) analysis. The X-ray powder diagram for the present invention was recorded with the MINIFLEX (Rigaku) instrument by depositing the sample on a static support with goniometric radius (distance between the X-ray tube and the support or, the equivalent, between the support and the detector) of 150 mm. Figure 8 shows an XRPD diffractogram of a sample of barnidipine hydrochloride, form I.

**Table 9. XRPD: list of characteristic peaks**

| **2Θ [°]** | **d-value [Å]** | **Intensity** | **I/Io** |
|---|---|---|---|
| 6.26 | 14.11 | 1485 | 53 |
| 7.12 | 12.40 | 2605 | 93 |
| 9.14 | 9.67 | 548 | 20 |
| 9.72 | 9.09 | 1976 | 71 |
| 12.40 | 7.13 | 1170 | 42 |
| 14.14 | 6.26 | 624 | 23 |
| 15.22 | 5.82 | 1486 | 53 |
| 17.08 | 5.19 | 1415 | 51 |
| 18.20 | 4.87 | 1103 | 40 |
| 18.80 | 4.71 | 478 | 17 |
| 19.48 | 4.55 | 1329 | 48 |
| 20.30 | 4.37 | 445 | 16 |
| 21.20 | 4.19 | 764 | 28 |
| 21.68 | 4.09 | 2727 | 97 |
| 22.04 | 4.03 | 880 | 32 |
| 22.70 | 3.91 | 1377 | 49 |
| 23.58 | 3.77 | 1115 | 40 |
| 23.92 | 3.72 | 513 | 19 |
| 24.72 | 3.60 | 606 | 22 |
| 25.42 | 3.50 | 871 | 31 |
| 26.28 | 3.39 | 709 | 26 |
| 26.84 | 3.32 | 596 | 22 |
| 27.36 | 3.25 | 2099 | 75 |
| 27.82 | 3.20 | 2813 | 100 |
| 28.34 | 3.15 | 848 | 31 |
| 28.68 | 3.11 | 892 | 32 |
| 29.44 | 3.03 | 904 | 33 |
| 29.86 | 2.99 | 692 | 25 |
| 30.28 | 2.95 | 914 | 33 |
| 31.04 | 2.88 | 517 | 19 |
| 31.72 | 2.82 | 680 | 25 |
| 32.54 | 2.75 | 549 | 20 |
| 33.92 | 2.64 | 767 | 28 |
| 35.10 | 2.55 | 550 | 20 |
| 35.80 | 2.50 | 640 | 23 |
| 36.56 | 2.45 | 478 | 17 |
| 37.10 | 2.42 | 480 | 18 |
| 37.76 | 2.38 | 491 | 18 |
| 38.58 | 2.33 | 625 | 23 |
| 39.72 | 2.27 | 595 | 22 |

In Table 9, the value "2Θ [°]" represents the angle of diffraction in degrees, and "d-value [Å]" represents the specific intervals between the lattice planes in Å.

Form I of barnidipine hydrochloride, used for the purpose of the present invention as comparator, is therefore characterised by an X-ray powder diagram with characteristic values of 2Θ = 6.3±0.2°, 7.1±0.2°, 9.1±0.2°, 9.7±0.2°, 12.4±0.2°, 14.1±0.2°, 15.2±0.2°, 17.1±0.2°, 18.2±0.2°, 18.8±0.2°, 19.5±0.2°, 20.3±0.2°, 21.2±0.2°, 21.7±0.2°, 22.0±0.2°, 22.7±0.2°, 23.6±0.2°, 23.9±0.2°, 24.7±0.2°, 25.4±0.2°, 26.3±0.2°, 26.8±0.2°, 27.4±0.2°, 27.8±0.2°, 28.3±0.2°, 28.7±0.2°, 29.4±0.2°, 29.9±0.2°, 30.3±0.2°, 31.0±0.2°, 31.7±0.2°, 32.5±0.2°, 33.9±0.2°, 35.1±0.2°, 35.8±0.2°, 36.6±0.2°, 37.1±0.2°, 37.8±0.2°, 38.6±0.2° and 39.7±0.2° and d-value = 14.11 Å, 12.40 Å, 9.67 Å, 9.09 Å, 6.26 Å, 4.87 Å, 4.19 Å, 3.72 Å and 3.03 Å *inter alia*.

On the basis of the published crystallography data (JMC, 1996, 29, 2504-2511) relating to barnidipine hydrochloride produced according to the state of the art, an X-ray powder simulation was conducted with PowderCell (for Windows, version 2.4) (W. Kraus and G. Nolze, PowderCell for Windows - Version 2.4 -Structure Visualisation/Manipulation, Powder Pattern Calculation and Profile Fitting, (2000) Federal Institute for Materials Research and Testing, Berlin, Germany). The diffractogram obtained from that simulation, overlaid on the one obtained experimentally with our sample of barnidipine hydrochloride form I, showed a very high correlation (Figure 9). The small variations observed can be attributed to the low resolution in the experimental acquisition compared with that of the diffraction lines calculated.

This clearly demonstrates that the product we obtained by following the indications in the literature corresponds to the form known to the prior art, herein called form I.

This is confirmed by the fact that the corresponding crystalline cell was extracted from the X-ray powder diffractogram of barnidipine hydrochloride form I that we obtained experimentally (Figure 8) using the programs DASH (W.I.F. David, K Shankland, J. van de Streek, E. Pidcock, W.D.S. Motherwell, J.C. Cole, DASH: a program for Crystal Structure Determination from Powder Diffraction Data. J. Appl. Cryst., 2006, 39, 910-915) and McMaille (Monte Carlo and grid search powder pattern indexing program, A. Le Bail, Powder Diffraction 19 (2004) 249-254). DASH was used to select the most significant peaks in the 2-theta 5-30° range in the X-ray powder diffractogram, and McMaille to determine the elemental cell. The cell extracted with the McMaille programme proved to belong to the monoclinic crystal system with the following dimensions: a = 14.3074 Å, b = 7.5624 Å, c = 12.5271 Å, V = 1352 Å³, beta = 93.92°. Alternatively, the same cell was extracted by the DASH programme and proved to belong to the monoclinic crystal system, spatial group P2₁, with the following dimensions: a = 14.2955 Å, b = 7.5641 Å, c = 12.5229 Å, V = 1351 Å³, beta = 93.87°.

The data obtained, reported above, agree with those reported in the literature for form I. In J. Med Chem, 1986, 29, 2504-2511 it is reported that form I of barnidipine hydrochloride has spatial group P2₁ and the following dimensions: a = 14.429(4) Å, b = 7.584(2) Å, c = 12.661(4) Å, V = 1383 Å³, Z = 2, d_{calc}(g/cm³) = 1.27.

It is also evident that the form I of barnidipine hydrochloride obtained differs considerably from the other form we obtained, described as form II, in terms of all the chemico-physical properties analysed (DSC, IR, Raman and XRPD diffractogram).

### Examples of synthesis

The following are non-limiting examples of the invention

### Example 1 (crude barnidipine hydrochloride)

Crude barnidipine malate (II) (247g, 0.39 mmol, 1.0 eq.) is placed in a 4-necked 3 L flask fitted with thermometer, mechanical stirrer and coolant, and ethanol (250 mL) is added. A solution of HCl in ethanol, previously prepared by bubbling gaseous HCl (15.8 g, 0.434 mmol, 1.1 eq.) in ethanol (1.0 L), is added to the suspension thus obtained, placed under stirring at 15±5°C. The resulting suspension is maintained under constant stirring at 15±5°C for 4h. The system is then cooled in an ice bath and the solid is isolated by filtration through a porous septum, washing with ethanol (300 mL). 187 g (90%) of crude barnidipine hydrochloride is obtained.

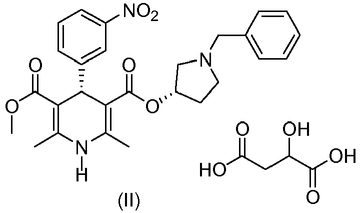

### Example 2 (Barnidipine hydrochloride form II, from anhydrous ethanol)

Crude barnidipine hydrochloride (50 g, 94.6 mmol) is placed in a 2 L flask, and anhydrous ethanol (1 L) is added. The resulting suspension, placed under stirring, is heated to the reflux temperature of the solvent. The solid product completely dissolves. Part of the solvent is removed by distillation (600 mL). The start of precipitation is observed. The mixture is refluxed under stirring for 12 h. The yellow suspension thus obtained is cooled to ambient temperature in 2.5h. The yellow solid obtained is isolated by filtration through a porous septum and washed with ethanol (100 mL). Yield: 45.5 g (91%). HPLC purity: >99.9%. IR (cm⁻¹): 3382, 2948, 1697, 1665, 1641, 1517, 1470, 1425, 1355, 1342, 1292, 1214, 1191, 1113, 1060 and 1021 *inter alia.*

Barnidipine hydrochloride form II.

Note: If denatured ethanol containing 3-6% methanol and 1-3% cyclohexane is used, NO variation is found.

### Example 3 (barnidipine hydrochloride form II, from 95% ethanol)

Crude barnidipine hydrochloride (50 g, 94.6 mmol) is placed in a 1L flask, and 95% ethanol (750 mL) is added. The resulting suspension, placed under stirring, is heated to the reflux temperature of the solvent until total dissolution takes place. Part of the solvent is removed by distillation (550 mL). The start of precipitation is observed. The system is refluxed under stirring for 11 h. The yellow suspension thus obtained is cooled to ambient temperature in 2.5 h. The yellow solid obtained is isolated by filtration through a porous septum, washing with ethanol (100 mL). Yield: 45 g (90%). HPLC purity: >99.9%. IR (cm⁻¹): 3382, 2948, 1697, 1665, 1641, 1517, 1470, 1425, 1355, 1342, 1292, 1214, 1191, 1113, 1060 and 1021 *inter alia.*

Barnidipine hydrochloride form II.

### Example 4 (barnidipine hydrochloride form II, from acetone/water)

Crude barnidipine hydrochloride (10 g) is placed in a 250 mL flask, and acetone (50 mL) is added. The resulting suspension, placed under stirring, is heated to the reflux temperature of the solvent. Water (8.0 mL) is added, and total dissolution is observed. The solution thus obtained is refluxed for 30 min. Acetone (60 mL) is added, and the precipitation of the product is observed. The system is then left under reflux and stirring for 12 h. The yellow suspension thus obtained is cooled to ambient temperature for 2.5 h. The solid obtained is isolated by filtration through a porous septum and washed with ethanol (15 mL). Yield: 7.5 g (75%). HPLC purity: >99.9%. IR (cm⁻¹): 3382, 2948, 1697, 1665, 1641, 1517, 1470, 1425, 1355, 1342, 1292, 1214, 1191, 1113, 1060 and 1021 *inter alia.*

### Barnidipine hydrochloride form II

### Example 5 (barnidipine hydrochloride form I, from anhydrous ethanol) - comparison product

Crude barnidipine hydrochloride (50 g, 94.6 mmol) is placed in a 2 L flask, and anhydrous ethanol (850 L) is added. The resulting suspension, placed under stirring, is heated to the reflux temperature of the solvent. The suspension dissolves completely. The solution obtained, maintained under stirring, is cooled slowly to ambient temperature in 2 h and left under stirring at that temperature for a further 2 h. Finally, the system is cooled in an ice bath and the precipitate is isolated by filtration through a porous septum, washing with ethanol (100 mL). Yield: 45.5 g (91%). HPLC purity: >99.9%. IR (cm⁻¹): 3170, 2949, 2480, 1715, 1649, 1529, 1429, 1346, 1307, 1299, 1270, 1094, 907 and 752 cm⁻¹ *inter alia.*

Barnidipine hydrochloride form I.

### Example 6 (barnidipine hydrochloride form I, from 95% ethanol) - comparison product

Crude barnidipine hydrochloride (50 g, 94.6 mmol) is placed in a 1 L flask, and a 95% ethanol mixture (550 L) is added. The resulting suspension, placed under stirring, is heated to the reflux temperature of the solvent. The suspension dissolves completely. The solution obtained, maintained under stirring, is cooled to 55-60°C in 1 h. Precipitation of the product is observed. The resulting suspension is left under stirring at 55-60°C for 2 h, and then cooled to ambient temperature in 2 h. The system is then cooled in an ice bath and the precipitate is isolated by filtration through a porous septum, washing with ethanol (100 mL). Yield: 45.5 g (91%). HPLC purity: >99.9%. IR (cm⁻¹): 3170, 2949, 2480, 1715, 1649, 1529, 1429, 1346, 1307, 1299, 1270, 1094, 907 and 752 cm⁻¹ *inter alia.*

Barnidipine hydrochloride form I.

### Example 7 (barnidipine hydrochloride form I, from acetone/water) - comparison product

Crude barnidipine hydrochloride (10 g) is placed in a 250 mL flask, and acetone (50 mL) is added. The resulting suspension, placed under stirring, is heated to the reflux temperature of the solvent. Water (8.0 mL) is added, and total dissolution is observed. The resulting solution is refluxed for 30 min. Acetone (50 mL) is added, and precipitation of the product is observed. The suspension obtained is cooled to 50°C, left under stirring for 2 h, then cooled to ambient temperature in 2.5 h. The solid obtained is isolated by filtration through a porous septum, washing with ethanol (15 mL). Yield: 7.5 g (75%). HPLC purity: >99.9%. IR (cm⁻¹): 3170, 2949, 2480, 1715, 1649, 1529, 1429, 1346, 1307, 1299, 1270, 1094, 907 and 752 *inter alia.*

Barnidipine hydrochloride form I.

### Example 8 (barnidipine hydrochloride form I, from methanol) - comparison product

Crude barnidipine hydrochloride (10 g) is placed in a 250 mL flask. and methanol (70 mL) is added. The resulting suspension, placed under stirring, is heated to the reflux temperature of the solvent and maintained until total dissolution is observed. The resulting solution is cooled and left under stirring for 3 h. The solid obtained is isolated by filtration through a porous septum, washing with ethanol (15 mL). Yield: 7.5 g (75%). HPLC purity: >99.9%. IR (cm⁻¹): 3170, 2949, 2480, 1715, 1649, 1529, 1429, 1346, 1307, 1299, 1270, 1094, 907 and 752 *inter alia.*

Barnidipine hydrochloride form I.

### Example 9 (barnidipine hydrochloride, conversion of form I to form II)

Barnidipine hydrochloride form I (50 g, 94.6 mmol) is placed in a 1 L flask, and 95% ethanol (750 L) is added. The resulting suspension, placed under stirring, is heated to the reflux temperature of the solvent. Total dissolution is observed. Part of the solvent is removed by distillation (550 mL). The start of precipitation is observed. The mixture is refluxed for 12 h. The yellow suspension thus obtained is cooled to ambient temperature in 2.5h. The yellow solid obtained is isolated by filtration through a porous septum, washing with ethanol (100 mL). Yield: 45 g (90%). HPLC purity: >99.9%. IR (cm⁻¹): 3382, 2948, 1697, 1665, 1641, 1517, 1470, 1425, 1355, 1342, 1292, 1214, 1191, 1113, 1060 and 1021 *inter alia.*

Barnidipine hydrochloride form II.

In the numerical values given in the text of the present application, the decimal point is used instead of the decimal comma.

## Claims

1. A crystalline form, named form II, of barnidipine hydrochloride (S,S) of formula (I) 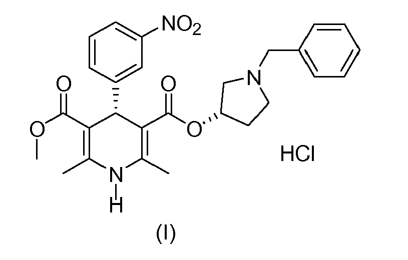 **characterised by** an orthorhombic elemental cell having the following dimensions: a = 7.185(2) Å, b = 16.123(4) Å, c = 22.571(6) Å, V = 2614.7(12) Å³, determined by structural single-crystal X-ray analysis.

2. The crystalline form of barnidipine hydrochloride according to claim 1, further **characterised by** an X-ray powder diffraction pattern having main peaks, expressed in 2-theta degrees [°], at 8.0±0.2, 11.2±0.2, 13.1±0.2, 13.7±0.2 and 15.9±0.2.

3. The crystalline form of barnidipine hydrochloride according to claims 1 and 2, further **characterised by** an X-ray powder diffraction pattern also having characteristic peaks expressed in 2-theta degrees [°] at 18.5±0.2, 20.2±0.2, 26.0±0.2 and 28.9±0.2.

4. The crystalline form of barnidipine hydrochloride according to claims 1-3, further **characterised by** an X-ray powder diffraction pattern also having characteristic peaks expressed in 2-theta degrees [°] at 16.8±0.2, 20.5±0.2, 24.1±0.2 and 30.1±0.2.

5. The crystalline form of barnidipine hydrochloride according to claims 1-4, **characterised by** an IR spectrum with characteristic peaks at 3382, 2948, 1697, 1665, 1641, 1517, 1470, 1425, 1342, 1292, 1214, 1191, 1060 and 1021 cm⁻¹.

6. The crystalline form of barnidipine hydrochloride according to claims 1-5, **characterised by** a DSC diagram having a characteristic endothermic peak at 229.2°C.

7. The crystalline form of barnidipine hydrochloride according to claims 1-6, **characterised by** a Raman spectrum with characteristic peaks at 1699, 1642, 1622, 1517, 1342, 1220, 1116, 949 and 849 cm⁻¹ Raman shift.

8. A process to obtain barnidipine hydrochloride form II, according to claim 1, by means of recrystallisation of the crude hydrochloride in a solvent or a mixture of solvents **characterised in that**, after the solubilisation of the crude hydrochloride in the solvent or mixture of solvents at boiling point:
a) part of the solvent is evaporated off by distillation until incipient crystallisation of a precipitate while heating
b) the suspension is then kept heated, at a temperature between 60° and 90°C, for a time of 3 to 20 hours
c) the suspension is then allowed to cool to room temperature during a time ranging from 1 to 4 hours in order to complete crystallisation.

9. A process according to claim 8 wherein, in step b), the temperature is kept between 70° and 85°C.

10. A process according to claim 8 wherein, in step b), the suspension is kept hot for 5 to 18 hours.

11. A process according to claim 10 wherein, in step b), the suspension is kept hot for 8 to 14 hours.

12. A process according to claim 8 wherein the solvent or mixture of solvents is selected from ethanol, isopropyl alcohol, propanol, acetone, methyl ethyl ketone, acetonitrile or mixtures thereof, or mixtures between them and water.

13. A process according to claim 12 wherein the solvent or mixture of solvents is selected from ethanol, acetone, ethanol/water and acetone/water.

14. A process according to claim 13 wherein the solvent is ethanol.

15. A process according to claim 8 wherein, in order to complete the crystallisation, the suspension is cooled and left at room temperature for 1-4 hours before collecting the final barnidipine hydrochloride form II by filtration.

16. A process according to claim 8 wherein the crude barnidipine hydrochloride is obtained starting from the corresponding malate salt by anion exchange of the salt *in situ*, with hydrochloric acid in acetone.

## Patentansprüche

1. Kristalline Form, Form II genannt, von Barnidipin-Hydrochlorid (S,S) der Formel (I): 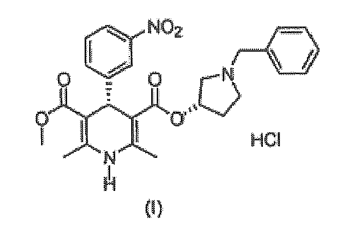 **gekennzeichnet durch** eine orthorhombische Elementarzelle mit den folgenden Abmessungen: a = 7,185(2) Å, b = 16,123(4) Å, c = 22,571(6) Å, V = 2614,7(12) Å³, bestimmt **durch** Einkristall-Röntgenstrukturanalyse.

2. Kristalline Form von Barnidipin-Hydrochlorid gemäß Anspruch 1, weiterhin **gekennzeichnet durch** ein Röntgenpulverbeugungsmuster, das als 2 Theta in Grad [°] ausgedrückte Hauptsignale bei 8,0 ± 0,2, 11,2 ± 0,2, 13,1 ± 0,2, 13,7 ± 0,2 und 15,9 ± 0,2 aufweist.

3. Kristalline Form von Barnidipin-Hydrochlorid gemäß Anspruch 1 und 2, weiterhin **gekennzeichnet durch** ein Röntgenpulverbeugungsmuster, das ebenfalls als 2 Theta in Grad [°] ausgedrückte charakteristische Signale bei 18,5 ± 0,2, 20,2 ± 0,2, 26,0 ± 0,2 und 28,9 ± 0,2 aufweist.

4. Kristalline Form von Barnidipin-Hydrochlorid gemäß Anspruch 1 bis 3, weiterhin **gekennzeichnet durch** ein Röntgenpulverbeugungsmuster, das ebenfalls als 2 Theta in Grad [°] ausgedrückte charakteristische Signale bei 16,8 ± 0,2, 20,5 ± 0,2, 24,1 ± 0,2 und 30,1 ± 0,2 aufweist.

5. Kristalline Form von Barnidipin-Hydrochlorid gemäß Anspruch 1 bis 4, **gekennzeichnet durch** ein IR-Spektrum mit charakteristischen Peaks bei 3382, 2948, 1697, 1665, 1641, 1517, 1470, 1425, 1342, 1292, 1214, 1191, 1060 und 1021 cm⁻¹.

6. Kristalline Form von Barnidipin-Hydrochlorid gemäß Anspruch 1 bis 5, **gekennzeichnet durch** ein DSC-Diagramm mit einem charakteristischen endothermen Peak bei 229,2 °C.

7. Kristalline Form von Barnidipin-Hydrochlorid gemäß Anspruch 1 bis 6, **gekennzeichnet durch** ein Raman-Spektrum mit charakteristischen Peaks bei 1699, 1642, 1622, 1517, 1342, 1220, 1116, 949 und 849 cm⁻¹ Raman-Verschiebung.

8. Verfahren zur Gewinnung von Barnidipin-Hydrochlorid Form II gemäß Anspruch 1 durch Umkristallisieren des rohen Hydrochlorids in einem Lösungsmittel oder Lösungsmittelgemisch, **dadurch gekennzeichnet, dass** nach der Auflösung des rohen Hydrochlorids in dem Lösungsmittel oder Lösungsmittelgemisch am Siedepunkt:
a) ein Teil des Lösungsmittels durch Destillation verdampft wird, bis die Kristallisation eines Niederschlags während des Erhitzens einsetzt;
b) die Suspension dann über einen Zeitraum von 3 bis 20 Stunden bei einer Temperatur zwischen 60 und 90 °C im erhitzten Zustand gehalten wird;
c) die Suspension dann während einer Zeit im Bereich von 1 bis 4 Stunden auf Raumtemperatur abkühlen gelassen wird, um die Kristallisation zu vollenden.

9. Verfahren gemäß Anspruch 8, wobei in Schritt b) die Temperatur zwischen 70 und 85 °C gehalten wird.

10. Verfahren gemäß Anspruch 8, wobei in Schritt b) die Suspension 5 bis 18 Stunden lang heiß gehalten wird.

11. Verfahren gemäß Anspruch 10, wobei in Schritt b) die Suspension 8 bis 14 Stunden lang heiß gehalten wird.

12. Verfahren gemäß Anspruch 8, wobei das Lösungsmittel oder Lösungsmittelgemisch aus Ethanol, Isopropylalkohol, Propanol, Aceton, Methylethylketon, Acetonitril oder Gemischen davon oder Gemischen von diesen mit Wasser ausgewählt ist.

13. Verfahren gemäß Anspruch 12, wobei das Lösungsmittel oder Lösungsmittelgemisch aus Ethanol, Aceton, Ethanol/Wasser und Aceton/Wasser ausgewählt ist.

14. Verfahren gemäß Anspruch 13, wobei es sich bei dem Lösungsmittel um Ethanol handelt.

15. Verfahren gemäß Anspruch 8, wobei zur Vollendung der Kristallisation die Suspension abgekühlt und 1-4 Stunden lang bei Raumtemperatur stehen gelassen wird, bevor das endgültige Barnidipin-Hydrochlorid Form II durch Filtration aufgefangen wird.

16. Verfahren gemäß Anspruch 8, wobei das rohe Barnidipin-Hydrochlorid ausgehend von dem entsprechenden Malatsalz durch Anionenaustausch des Salzes in situ mit Chlorwasserstoffsäure in Aceton erhalten wird.

## Revendications

1. Forme cristalline, désignée par l'appellation « forme II », du chlorhydrate de barnidipine (S,S) répondant à la formule (I) 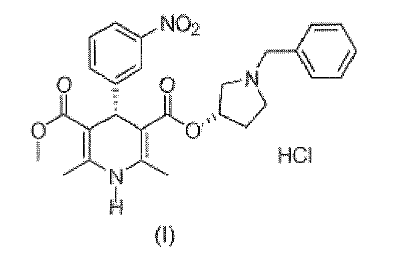 **caractérisée par** une cellule élémentaire orthorhombique possédant les dimensions suivantes : a = 7,185(2) Å, b = 16,123(4) Å, c = 22,571(6) Å, V = 2614,7(12) Å³, déterminées par une analyse structurelle par rayons X sur monocristaux.

2. Forme cristalline du chlorhydrate de barnidipine selon la revendication 1, **caractérisée en outre par** un diagramme de diffraction de rayons X sur poudres possédant des pics principaux, exprimés en degrés [°] 2θ, à 8,0 ± 0,2, 11,2 ± 0,2, 13,1 ± 0,2, 13,7 ± 0,2 et 15,9 ± 0,2.

3. Forme cristalline du chlorhydrate de barnidipine selon les revendications 1 et 2, **caractérisée en outre par** un diagramme de diffraction de rayons X sur poudres possédant également des pics caractéristiques, exprimés en degrés [°] 2θ, à 18,5 ± 0,2, 20,2 ± 0,2, 26,0 ± 0,2 et 28,9 ± 0,2.

4. Forme cristalline du chlorhydrate de barnidipine selon les revendications 1 à 3, **caractérisée en outre par** un diagramme de diffraction de rayons X sur poudres possédant également des pics caractéristiques, exprimés en degrés [°] 2θ, à 16,8 ± 0,2, 20,5 ± 0,2, 24,1 ± 0,2 et 30,1 ± 0,2.

5. Forme cristalline du chlorhydrate de barnidipine selon les revendications 1 à 4, **caractérisée par** un spectre infrarouge comprenant des pics caractéristiques à 3382, 2948, 1697, 1665, 1641, 1517, 1470, 1425, 1342, 1292, 1214, 1191, 1060 et 1021 cm⁻¹.

6. Forme cristalline du chlorhydrate de barnidipine selon les revendications 1 à 5, **caractérisée par** un diagramme DSC possédant un pic endothermique caractéristique à 229,2 °C.

7. Forme cristalline du chlorhydrate de barnidipine selon les revendications 1 à 6, **caractérisée par** un spectre Raman comprenant des pics caractéristiques à des décalages Raman de 1699, 1642, 1622, 1517, 1342, 1220, 1116, 949 et 849 cm⁻¹.

8. Procédé pour obtenir la forme II du chlorhydrate de barnidipine selon la revendication 1 au moyen d'une recristallisation du chlorhydrate brut dans un solvant ou dans un mélange de solvants, **caractérisé en ce que**, après la solubilisation du chlorhydrate brut dans le solvant ou dans le mélange de solvants au point d'ébullition :
a) on évapore une partie du solvant par distillation jusqu'à la cristallisation naissante d'un précipité, tandis que l'on chauffe ;
b) on maintient la suspension à l'état chauffé, à une température entre 60° et 90 °C pendant un laps de temps de 3 à 20 heures ;
c) on laisse ensuite refroidir la suspension jusqu'à la température ambiante pendant un laps de temps qui se situe dans la plage de 1 à 4 heures dans le but d'amener la cristallisation à son terme.

9. Procédé selon la revendication 8, dans lequel, à l'étape b), on maintient la température entre 70° et 85 °C.

10. Procédé selon la revendication 8, dans lequel, à l'étape b), on maintient la suspension chaude pendant un laps de temps de 5 à 18 heures.

11. Procédé selon la revendication 10, dans lequel, à l'étape b), on maintient la suspension chaude pendant un laps de temps de 8 à 14 heures.

12. Procédé selon la revendication 8, dans lequel le solvant ou le mélange de solvants est choisi parmi l'éthanol, l'alcool isopropylique, le propanol, l'acétone, la méthyléthylcétone, l'acétonitrile ou leurs mélanges ou encore des mélanges d'entre eux et d'eau.

13. Procédé selon la revendication 12, dans lequel le solvant ou le mélange de solvants est choisi parmi l'éthanol, l'acétone, de l'éthanol/eau et de l'acétone/eau.

14. Procédé selon la revendication 13, dans lequel le solvant est l'éthanol.

15. Procédé selon la revendication 8, dans lequel, dans le but d'amener la cristallisation à son terme, on refroidit la suspension et on l'abandonne à la température ambiante pendant un laps de temps de 1 à 4 heures avant de récolter la forme Il finale du chlorhydrate de barnidipine par filtration.

16. Procédé selon la revendication 8, dans lequel on obtient le chlorhydrate brut de barnidipine à partir du sel malate correspondant par échange d'anions du sel *in situ* avec de l'acide chlorhydrique dans de l'acétone.
